# EUROPEAN PATENT APPLICATION

(11) **EP 1 867 972 A1**
(43) Date of publication of application: **19.12.2007**
(21) Application number: 06729015.5
(22) Date of filing: 14.03.2006
(51) Int. Cl.: G01N 1/10, G01N 33/48

(54) **SAMPLING LIQUID CONTAINER**

(30) Priority: 14.03.2005 JP 2005070840; 29.07.2005 JP 2005220025; 07.09.2005 JP 2005259045; 06.01.2006 JP 2006001557
(71) Applicant: NIPRO CORPORATION, Kita-ku, Osaka-shi, Osaka-fu, 531-8510 (JP)
(72) Inventor: HASEGAWA, Mitsuru, ita-ku, Osaka-shi, Osaka, 5318510 (JP); ABE, Toshinori, ita-ku, Osaka-shi, Osaka, 5318510 (JP); TOMITA, Masakatsu, ita-ku, Osaka-shi, Osaka, 5318510 (JP); OHTANI, Tadashi, ita-ku, Osaka-shi, Osaka, 5318510 (JP); ISHIKAWA, Atsushi, ita-ku, Osaka-shi, Osaka, 5318510 (JP)
(74) Representative: Banzer, Hans-Jörg
(86) International application number: PCT/JP2006/304954
(87) International publication number: WO 2006/098297

(57) **Abstract**

There is provided a sampling liquid container that overcomes drawbacks of a user's contamination and infection risk by virus, and a cumbersome handling.

There is provided the sampling liquid container incliding a tubular container opening in its upper end and lower end, a stopper slidably inserted into the tubular container in order to provide a fluid-tight dissolving solution compartment in the tubular container, and a cap which is detachably attached to the upper end of the tubular container, and having an adapter fluid-tightly attached to the lower end of the tubular container, **characterized in that** an inner diameter of the lower end of the tubular container is slightly larger than an outer diameter of the stopper, the adapter accommodates the stopper pushed down when the dissolving solution compartment of the tubular container is pressed, a gap is formed between a lower end inner wall of the tubular container and an outer face of the stopper, and additionally a drip port is possessed in the lower end of the tubular container.

## Description

### BACKGROUND OF THE INVENTION

### 1. Field of the Invention

The present invention relates to a sampling liquid container for testing a bacterium such as virus. More detailedly, it relates to the sampling liquid container having a structure capable of preventing a technician from a risk in contamination and infection by the virus when the sampling liquid dripped to a reaction reagent container for detecting the virus such as influenza is prepared.

### 2. Prior Art

As one of virus antigen test such as influenza, in recent years, there is used an influenza antigen test kit in which a test can be performed in a bed side. Additionally, by the test kit there becomes possible to rapidly detect also a judgment on A-type/B-type of the influenza. In the test kit like this, there is generally utilized an immuno-chromatography method, a flow-through method, an EIA method, or the like. In either these methods, it is performed by dropping the sampling liquid prepared from a specimen previously collected from a patient to the reaction reagent container, e.g., a reaction reagent cassette. Concretely, after a mucus is sucked by a suction device from a nasal cavity or pharynx of the patient or it is wiped by a swab, it is dissolved by a dissolving solution to thereby prepare the sampling liquid, and this sampling liquid is dripped to the reaction reagent container.

As a container for preparing the sampling liquid like this, it is possible to enumerate one in which it is dripped by replacing a cap of a capped container previously filled with the dissolving solution with a drip port cap (JP-A-2004-109015 Gazette). For example, a specimen treatment liquid is first prepared in a squeeze tube, and next the specimen is treated by immersing the swab, which collected the specimen, to the specimen treatment liquid, thereby preparing the sampling liquid. Additionally, after the squeeze tube is fitted with a drip port cap (drip chip) and is reversed, and then sample is dripped to a specimen drip part of the reaction reagent container from the drip port cap (drip chip).

In the container of the above type, the sampling liquid container can be made directly a dripping container, and it is possible to drip, from a first drop, a liquid uniformly extracted, in which fluctuation in every specimen is small. Further, if a filter is attached to the drip port cap (drip chip), there is no fact that a clogging occurs even if there is contained a pus whose viscosity is high or a solid matter. However, in this case, in a case where the sampling liquid spatters peripherally by attaching the drip port cap (drip chip), a case where the drip port cap is small, and the like, a risk that the technician is infected by the virus becomes extremely high.

Further, in a case where an exclusive cap attached to the drip port does not exist, when discarding the container after the drip, the sampling liquid spreads in the discarded container, so that the risk of the infection becomes high also on the occasion of a waste disposal. Additionally, there follows such a cumbersome work that the same amount of drip port caps as the number of the specimens must be separately, previously prepared. Whereupon, a problem of the present invention is to provide a sampling liquid container in which there is solved a drawback of the user's contamination and infection risk by the virus, and a cumbersome handling.

Other problem of the present invention is to provide the above sampling liquid container whose taking-out from a stand rack of the containers is facilitated.

Further, another problem of the present invention is to provide the above sampling liquid container in which the preparation of a sample liquid can be simply performed.

Additionally, other problem of the present invention is to provide the above sampling liquid container in which the scattering of the sampling liquid can be prevented.

### SUMMARY OF THE INVENTION

That is, the present invention is a sampling liquid container including a tubular container opening in its upper end and lower end, a stopper slidably inserted into the tubular container in order to provide a fluid-tight dissolving solution compartment in the tubular container, and a cap for the opening, which is detachably attached to the upper end of the tubular container, and having an adapter fluid-tightly attached to the lower end of the tubular container, characterized in that an inner diameter of the lower end of the tubular container is slightly larger than an outer diameter of the stopper, the adapter accommodates the stopper pushed down when the dissolving solution compartment of the tubular container is pressed, a gap is created between a lower end inner wall of the tubular container and an outer face of the stopper, and additionally a drip port is fitted in the lower end of the tubular container.

Further, in the sampling liquid container of the present invention, a filter for trapping a high viscosity liquid is provided above the drip port while adjoining it. Additionally, in the sampling liquid container of the present invention, there is provided a cap for the drip port, which is detachably attached to the drip port.

In the sampling liquid container of the present invention, by the fact that the stopper is slidably inserted and additionally it has the drip port, it is possible to prevent the technician from the risk such as the contamination and the infection by the virus when preparing a drip liquid to the reaction reagent container. Further, it is also possible to reduce a complexity of operation.

Usually, the sampling liquid containers like this are juxtaposed in a paper-made stand rack like a test tube stand under a stood state, and this is accommodated in an outer box (packaging box) opening upward. When performing the test, a technician takes out the sampling liquid container by pinching an upper side part exposed from the stand rack of the containers.

At this time, the plural containers are regularly juxtaposed while being stood under an approximately adjoining state so as to be accommodated with a good packing, so that there is a problem that for the technician it is difficult to insert his/her fingers to the side part of the container even if attempted to pinch an upper part. In regard to this, in each maker there is performed a contrivance for giving a degree of freedom in an alignment of the containers such that some room is made in the side part when the containers are accommodated in the stand rack, by making such that an opening of the stand rack becomes slightly larger than an outer shape of a container barrel part, or accommodating the container in the stand rack so as to float by providing a flange in the side part of the container.

However, in a case where, after the specimen is collected, the container is stood again in the stand rack for a predetermined time in order to raise a test accuracy, a case where the same container is used in order to implement the tests of at least two kinds by the same container, a case where the container is stood again in the stand rack for waiting a retest, or the like, there is a fear that a spattered or oozed droplet of the sampling liquid, the specimen, or the like remains in the vicinity of a lower end of the cap, with which the technician's fingers are liable to contact, so that there is an issue that the risk of the technician's infection becomes high.

Further, when attaching/detaching the cap in order to introduce the specimen sample, the barrel part of the container is pinched by one hand and a side of the cap is pinched by another hand, so that similarly the technician has the risk of the infection.

Whereupon, as a result of earnestly performing various studies in order to solve the above problems, the present inventors reached to the present invention by thinking of agaping the risk of the infection by facilitating the taking-out of the container from the stand rack, and attaching/detaching the cap with a grip portion necessary for opening, which is separated from a boundary portion of the cap and the side part of a container main body at a mounting time (cap lower end), and by finding out providing the cap having a knob part in its top face to the sampling liquid container.

That is, the present invention is a sampling liquid container including a tubular container having in its upper end an opening part for introducing a specimen sample to a dissolving solution, and a cap for the opening, which is detachably attached to the opening part of the tubular container, characterized in that there is provided an auxiliary means taking out the container, which extends in the upper end and/or the lower end of the sampling liquid container.

In the present invention, by the fact that there is provided the auxiliary means taking out the container, which extends in the upper end and/or the lower end of the sampling liquid container, the technician can simply take out the sampling liquid container from an accommodation box, and it is possible to prevent the risk in the contamination and the infection by the virus.

In the sampling liquid container like this, in order to produce the sample liquid, the swab which collected the specimen is immersed to a specimen treatment liquid (dissolving solution) by one hand, and the specimen is extracted by rotating the swab by several revolutions while nip-pressing a cotton ball portion down from a periphery of the tubular container by fingers of the other hand. Next, the swab is taken out while squeezing a liquid out by strongly pressing the cotton ball portion down in a portion below a neck part of the tubular container and above a liquid level, and it is made the sample liquid.

However, from the fact that a mucus matter is contained in the specimen and it adheres to the swab, it is difficult to easily extract the specimen by the specimen treatment liquid only by rotating the swab by several revolutions while nip-pressing the cotton ball portion down from the periphery of the tubular container by fingers of the other hand.

Another problem of the present invention is to provide the sampling liquid container in which the preparation of the sample liquid can be simply performed.

That is, the present invention is a sampling liquid container having a tubular container opening in its upper end and lower end, a stopper slidably inserted into the tubular container in order to provide a fluid-tight dissolving solution compartment in the tubular container, a cap for the opening, which is detachably attached to the upper end of the tubular container, and an adapter whose upper end is fluid-tightly attached to the lower end of the tubular container and which possesses in its lower end a filter and a drip port, wherein an inner diameter of the lower end of the tubular container is slightly larger than an outer diameter of the stopper, the adapter accommodates the stopper pushed down when the dissolving solution compartment of the tubular container is pressed from an outside, and a gap is formed between a lower end inner wall of the tubular container and an outer face of the stopper, characterized in that an auxiliary means for promoting a dissolution or an extraction of a specimen sample in the dissolving solution compartment is provided in an inside of the tubular compartment.

In the present invention, by the fact that there is provided the auxiliary means for promoting the dissolution or the extraction of the specimen sample in the dissolving solution compartment, it is possible to simply perform a preparation of the sample liquid.

In the above sampling liquid container, the filter is provided in an inner cylinder part of the adapter while adjoining the drip port, and the filter removes a foreign matter in the sampling liquid and removes a viscous matter. However, because the stopper is normally formed by a material such as rubber or elastomer, since a blocking, i.e. , adhesion between the materials, occurs during a custody of the sampling liquid container, there is such an issue that there occurs the fact that the sampling liquid spatters like a water gun when performing a communication operation for dropping the sample to reaction reagent because, when the side part of the tubular container is squeezed for a liquid communication and a container internal pressure is rapidly raised, the blocking is suddenly released and the stopper slides by its power.

As a result of the fact that the present inventors variously, earnestly studied for preventing the spattering of the sampling liquid, it became possible to agap the issue like this by suitably selecting a pore diameter and a thickness of the filter, a pore diameter of the drip port, and the like.

That is, the present invention is a sampling liquid container having a tubular container opening in its upper end and lower end, a stopper slidably inserted into the tubular container in order to provide a fluid-tight dissolving solution compartment in the tubular container, and a cap for the opening, which is detachably attached to the upper end of the tubular container, and having an adapter fluid-tightly attached to the lower end of the tubular container, wherein an inner diameter of the lower end of the tubular container is slightly larger than an outer diameter of the stopper, the adapter accommodates the stopper pushed down when the dissolving solution compartment of the tubular container is pressed, a gap is formed between a lower end inner wall of the tubular container and an outer face of the stopper, and additionally a drip port is possessed in the lower end, characterized in that a filter is provided in an inner tube part of the adapter while adjoining the drip port, and the filter is a continuous porous body whose pore diameter is about 0.1 µm - about 20 µm, and whose thickness is about 1.0 mm - about 6.0 mm.

Further, it is desirable that the drip port has a hollow groove of about 0.5 mm - about 2 mm in bore diameter, and about 6 mm - about 15 mm in length and, additionally, it is desirable that a quantity of a liquid filled in the dissolving solution compartment is about 0.1 - 2.0 mL. Additionally, it is desirable that there is detachably provided a cap for the drip port to the drip port.

In the present invention, by selecting the pore diameter and the thickness of the filter, the bore diameter and the length of the drip port, and the liquid-filled quantity, it is possible to solve such an issue that the sampling liquid scatters like the water gun when the sample is dripped to the reaction reagent.

### ADVANTAGES OF THE INVENTION

In the sampling liquid container of the present invention, by slidably inserting the stopper and additionally having the drip port, it is possible to prevent the technician from the risk such as contamination and infection by the virus when preparing the drip liquid to the reaction reagent container. Further, it is also possible to reduce the complexity of the operation. Further, in the sampling liquid container of the present invention, by the fact that there is provided the auxiliary means taking out the container, which extends in the upper end and/or the lower end of the sampling liquid container, it is possible to facilitate the taking-out of the container from the stand rack, and by the fact that there is provided attaching/detaching the cap with the grip portion necessary for opening which is separated from a boundary portion of the cap and the side part of a container main body at a mounting time (cap lower end), it is possible to agap the risk of the infection. Additionally, in the present invention, by the fact that there is provided the auxiliary means for promoting the dissolution or the extraction of the specimen sample in the dissolving solution compartment, it is possible to simply perform the preparation of the sample liquid. Further, in the present invention, by selecting the pore diameter and the thickness of the filter, the bore diameter and the length of the drip port, and the liquid-filled quantity, it is possible to solve such an issue that the sampling liquid spatters like the water gun when the sample is dripped to the reaction reagent.

### BRIEF DESCRIPTION OF THE DRAWINGS

[Fig. 1] It is a longitudinal sectional view showing a state before use of one example of a sampling liquid container of the present invention.
[Fig. 2] It is a longitudinal sectional view showing a state at a dissolution adjustment time of the sampling liquid container shown in Fig. 1.
[Fig. 3] It is a schematic view showing a state at a drip time of the sampling liquid container shown in Fig. 1.
[Fig. 4] It is a longitudinal sectional view showing one example of the sampling liquid container of the present invention.
[Fig. 5] It is a front view of the sampling liquid container shown in Fig. 4.
[Fig. 6] It is a side view of the sampling liquid container shown in Fig. 4.
[Fig. 7] It is a top view of a knob part of the sampling liquid container shown in Fig. 4.
[Fig. 8] It is a schematic view taking out the sampling liquid container of the present invention from an accommodation box by a hand.
[Fig. 9] It is a longitudinal sectional view showing the state before use of other sampling liquid container of the present invention.
[Fig. 10] It is an A - A sectional view in Fig. 9.
[Fig. 11] It is a longitudinal sectional view showing the state at a dissolution preparation time of the sampling liquid container shown in Fig. 9.
[Fig. 12] It is a schematic view showing the state at the drip time of the sampling liquid container shown in Fig. 9.
[Fig. 13] It is a longitudinal sectional view showing other embodiment of an auxiliary means for promoting a dissolution or an extraction of a specimen sample of the present invention.
[Fig. 14] It is a longitudinal sectional view showing the state at the dissolution preparation time of the sampling liquid container shown in Fig. 4.
[Fig. 15] It is a schematic view showing the state at the drip time of the sampling liquid container shown in Fig. 4.

### DESCRIPTION OF REFERENCE NUMERALS

- 1: sampling liquid container
- 2: tubular container
- 3: stopper
- 4: cap for opening
- 41: knob of cap for opening
- 5: adapter
- 6: filter
- 7: dissolving solution compartment
- 8: drip port
- 9: drip port cap
- 91: knob of drip port cap

### BEST MODE FOR CARRYING OUT THE INVENTION

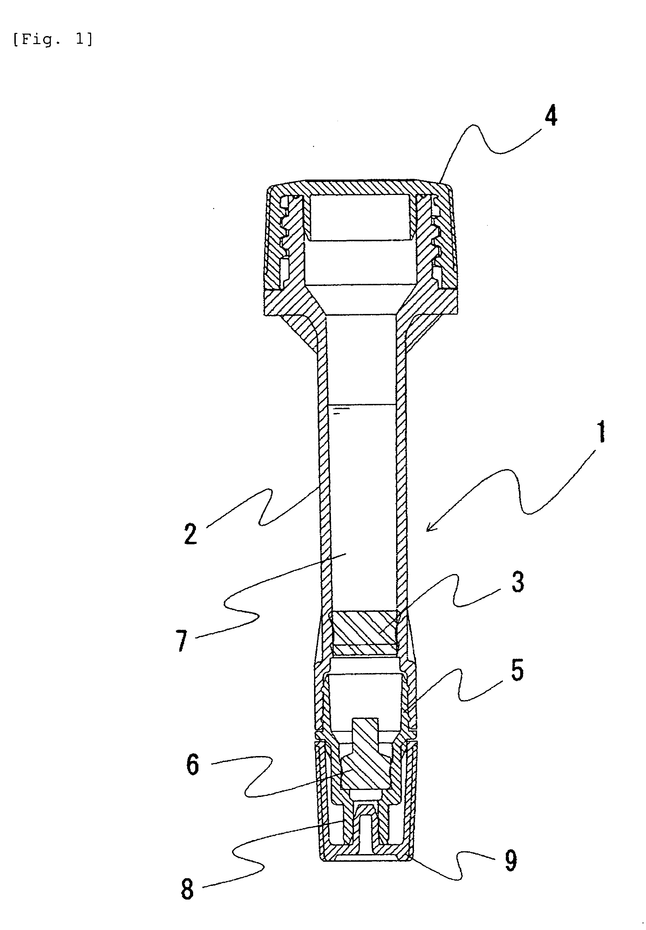

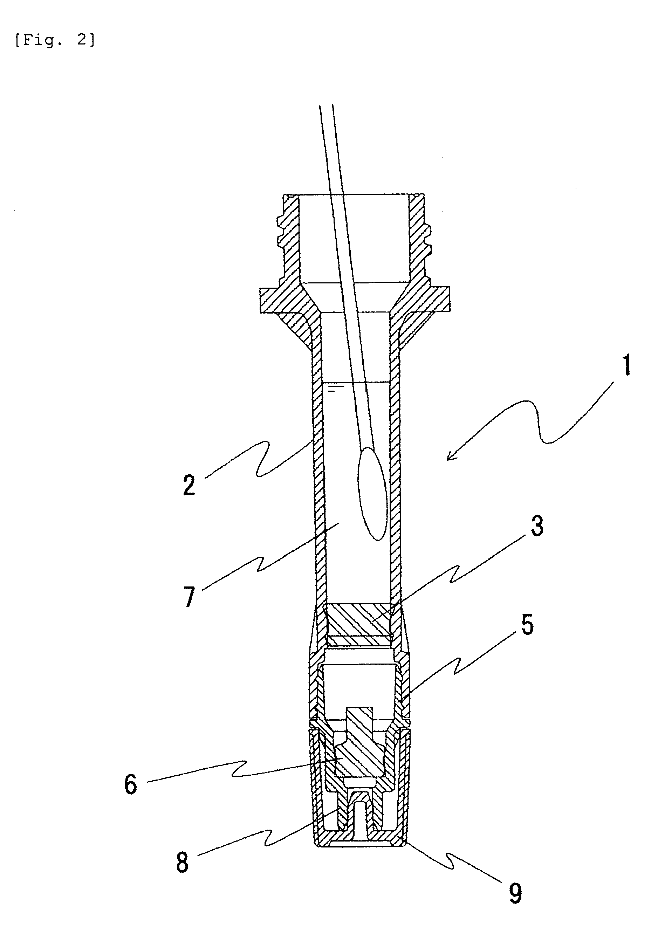

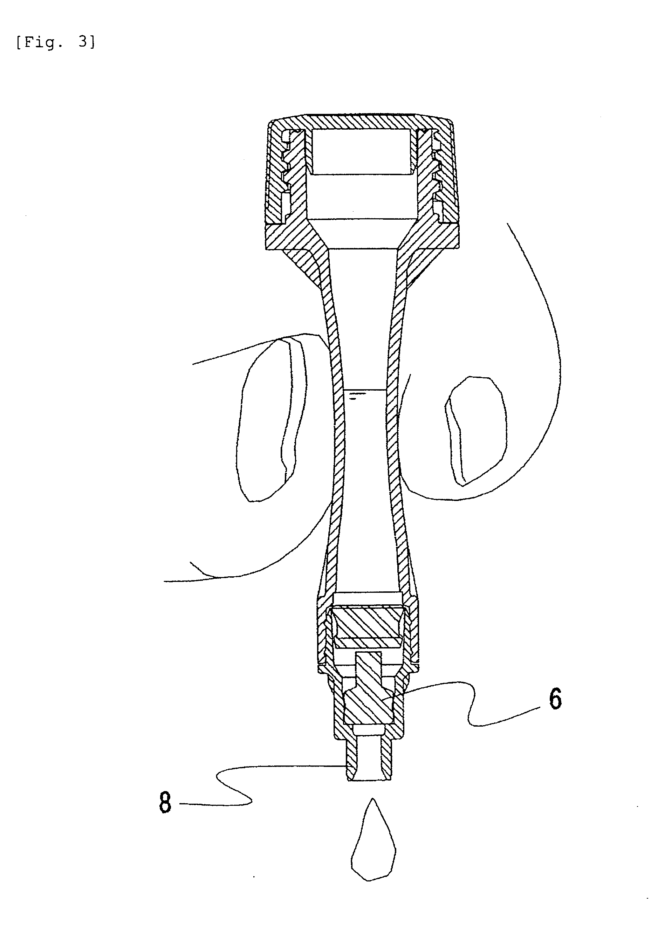

A sampling liquid container concerned with the present invention is concretely explained by using the drawings. Fig. 1 is a longitudinal sectional view of the sampling liquid container of the present invention, and Fig. 2 is a longitudinal sectional view showing a state in which a swab which collected a specimen is inserted into the sampling liquid container of the present invention from its opening part. Fig. 3 is a schematic view showing a state in which a dissolving solution compartment of a tubular container of the sampling liquid container of Fig. 1 is pressed from an outside, a stopper is pressed down, a sampling liquid passes through a gap between an inner face of the tubular container and an outer face of the stopper, and it drips from a drip port.

As shown in Fig. 1, a sampling liquid container 1 of the present invention has a tubular container 2 opening in its upper end and lower end, a stopper 3 slidably inserted into the tubular container 2 in order to provide a fluid-tight dissolving solution compartment 7 in the tubular container, and a cap 4 for the opening, which is detachably attached to the upper end of the tubular container 2, and has an adapter 5 fluid-tightly attached to the lower end of the tubular container 2.

The tubular container 2 has in its upper end an opening part with which the cap 4 for the opening can be normally mesh-engaged and, in its lower end, has a portion whose inner diameter is slightly larger than an outer diameter of the stopper 3. Further, the adapter 5 can be internally inserted into the lower end portion.

In the tubular container 2, the slidable stopper 3 is inserted in the vicinity of its lower end, and the cap 4 is detachably attached to its upper end. By the fact that the stopper 3 and the cap 4 are attached, the dissolving solution compartment 7 is formed in the tubular container 2. In the dissolving solution compartment 7, there is filled a liquid dissolving the specimen (nasal cavity wiping liquid, nasal cavity absorption liquid, pharynx wiping liquid, or the like). Usually, the dissolving solution is a water, and a surfactant is added in order to raise a reactivity with the specimen when the dissolving solution is dripped into the reaction reagent. Additionally, as an antiseptic, there is added sodium azide, or the like.

In order to push out the sampling liquid treated in the tubular container 2, there is not limited to the fact that a volume of the dissolving solution compartment is made relatively small compared to a dissolving solution quantity by pressing the tubular container 2 from an outside to thereby deform a tube part of the tubular container 2, and it may be pushed out by other physical means, or there may be performed such an operation as to raise an internal pressure by compressing a gas existing in the dissolving solution compartment.

Accordingly, it is desirable that the tubular container 2 has such a transparency that a state in the container can be confirmed and it does not become white even if repeatedly pressed, and a flexibility and, as a material like this, there is suitably used polyethylene, polypropylene, vinyl chloride resin, or the like.

Since the stopper 3 and the cap 4 are assembled while keeping a fluid-tightness of the dissolving solution compartment of the tubular container 2, there is no risk that the dissolving solution leaks from the tubular container 2 to the outside.

The stopper 3 has generally a shape of such a degree that there is no fact that it is distorted to thereby impair the fluid-tightness at a manufacturing time or at a long preservation time, desirably a cylindrical shape whose length (dimension) is small, and, before the specimen is added, it closely contacts with an inner wall of the tubular container 2. However, if the stopper 3 is slid and moved to a lower end direction of the tubular container 2, a gap is formed between it and the inner wall. There is made such that this gap is not closed even when the deformation of the dissolving solution compartment of the tubular container is restored.

As a material of the stopper 3, although there is enumerated an elastic material such as normal butyl rubber, halogenated butyl rubber, butadiene rubber, isoprene rubber, isobutylene rubber, or thermoplastic elastomer, usually the thermoplastic elastomer is used.

Further, for the purpose of smoothening a sliding, an annular rib may be provided in the stopper 3.

The cap 4 for the opening is detachably attached to the tubular container 2, and usually mesh-engaged. Further, in order to keep the fluid-tightness, it is usually provided a sleeve jointing with an opening end inner wall of the tubular container.

The adapter 5 is a tubular member connected to the lower end of the tubular container 2, accommodates the stopper 3 pushed down when the dissolving solution compartment 7 of the tubular container 2 is pressed, forms a gap between a lower end inner wall of the tubular container 2 and an outer face of the stopper 3, and additionally possesses in its lower end a drip port 8. Before the sampling liquid is prepared, usually only the air exists in the adapter 5.

If the adapter 5 is attached to the tubular container 2 while keeping the fluid-tightness, it may be fusion-bonded, or bonded by a solvent, or additionally bonded under a pressed-in state. As a material like this, there is used polyethylene, polypropylene, ABS resin, polystylene, BS resin, polycarbonate resin, or the like.

In a lumen of the adapter 5, a filter 6 is provided above the drip port 8 while adjoining it. The filter 6 is one which, in a case where the specimen collected from the patient is one having a high viscosity or contains the solid matter, has a bore diameter of such a degree that it is trapped such that the drip port does not clog. A filtration effective area and a coarse texture of the filter can be freely selected. As a material like this, besides polyethylene, polypropylene, fluorine-based resin, nylon resin, or the like, although there is used an inorganic material or the like, usually there is used a polyethylene sintered body.

To the drip port 8, there is provided a cap 9 for the drip port, which is detachably attached. The cap 9 for the drip port may be detachably attached to the drip port 8 and, as a material, there is used polyethylene, polypropylene, vinyl chloride resin, or the like. Further, it may be formed monolithically with the adapter 5. Additionally, in order that the plural sampling liquid containers can be stood in an exclusive container stand, a diameter of the exclusive container stand may be made a size approximately the same as a barrel diameter of the tubular container 2.

As shown in Fig. 2, when preparing the sampling liquid, first the cap 4 of the opening part is released under a state directed upward, a sampling rod, such as swab, to which there adheres the specimen collected from the patient is inserted into the dissolving compartment of the tubular container 2 from an upper end opening part, and the specimen is dissolution-extracted by the dissolving solution, thereby preparing the sampling liquid.

Next, as shown in Fig. 3, the cap 4 is attached again to the opening part of the tubular container 2 and, after the drip port 8 placed in a side opposite to the container 2 is directed to a reaction reagent container, if a barrel part of the tubular container 2, in which the dissolving solution compartment exists, is pressed, the stopper 3 advances downward by a pressure rise in an inside due to that pressing pressure, and moves into the adapter 5 and is accommodated. Between an outer face of the accommodated stopper 4 and an inner wall of the adapter 5, there is provided a gap through which the sampling liquid in the dissolving solution compartment 7 can pass and drip. Accordingly, the sampling liquid passes through this gap, is guided from the drip port 8 to the outside, and can drip to the reaction reagent container.

Other sampling liquid container concerned with the present invention is concretely explained by using the drawings.

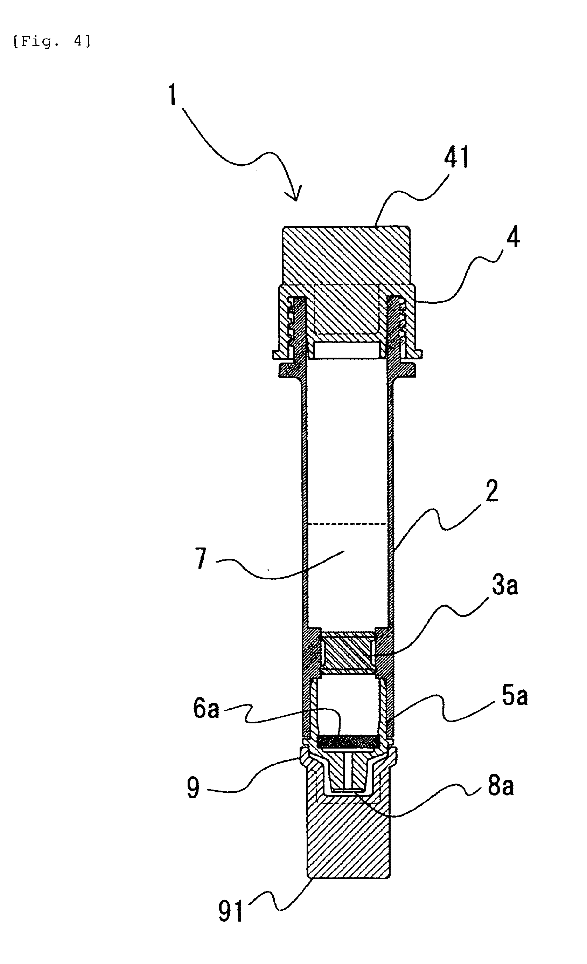

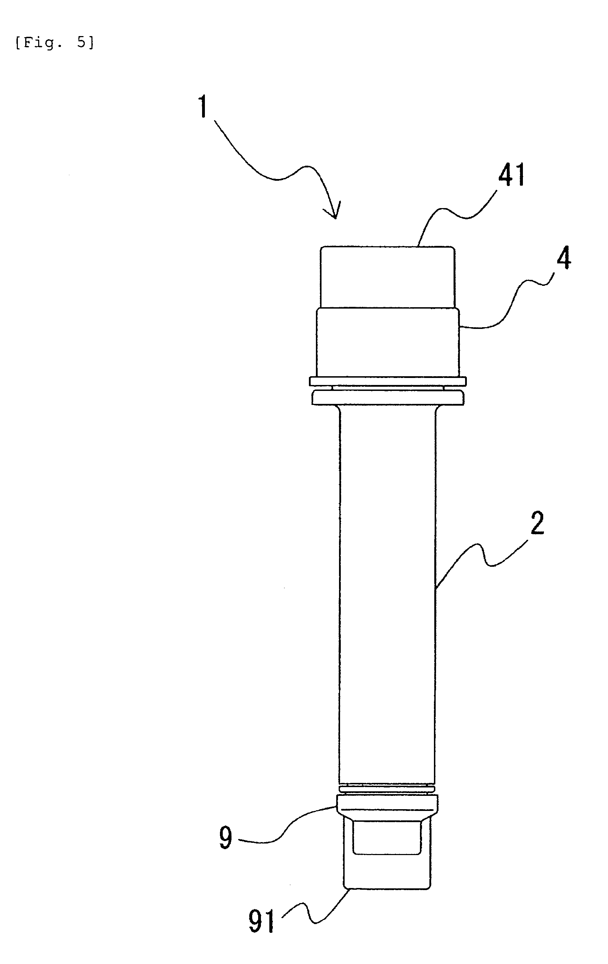

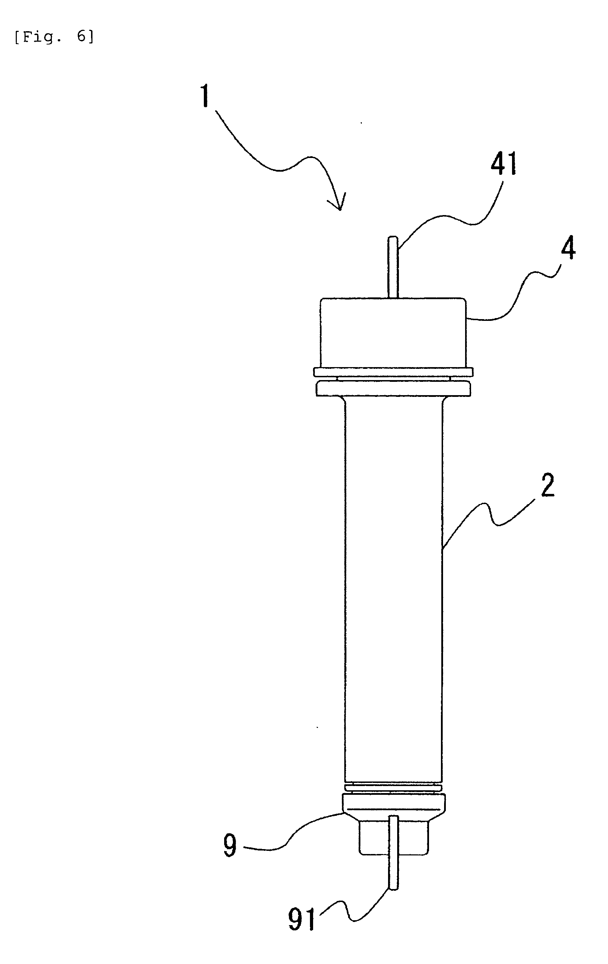

Fig. 4 is a longitudinal sectional view showing a state before use in one example of the sampling liquid container of the present invention, Fig. 5 is a front view showing the sampling liquid container of Fig. 4, and Fig. 6 is a side view.

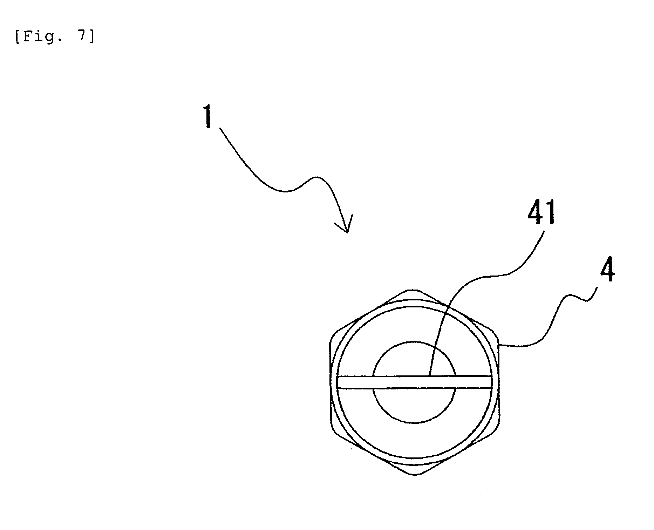

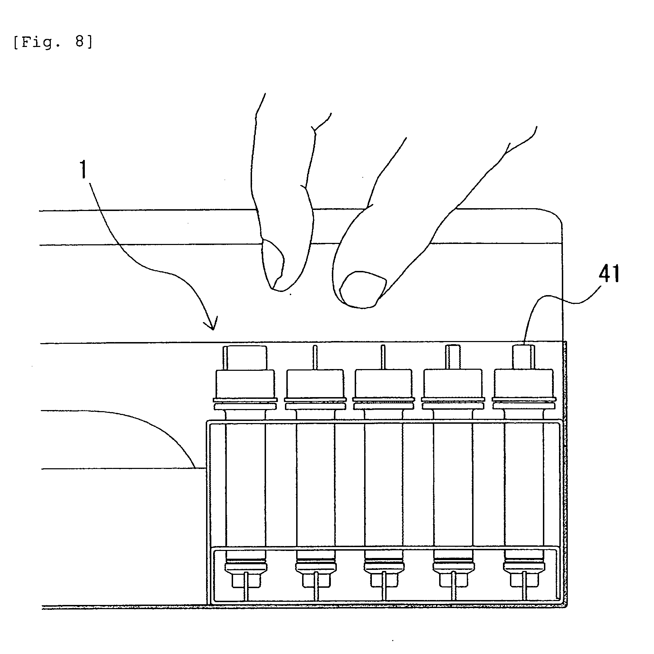

Fig. 7 is a top view of a knob part. Fig. 8 is a schematic view in which an technician is attempting to take out, from an accommodation box accommodating plural sampling liquid containers, the container by a hand.

As shown in Fig. 4, the sampling liquid container 1 of the present invention has the tubular container 2 opening in its upper end and lower end, a stopper (seal member) 3a slidably inserted into the tubular container 2 in order to provide the fluid-tight dissolving solution compartment 7 in the tubular container, and the cap 4 for the opening, which is detachably attached to the upper end of the tubular container 2, and has an adapter 5a fluid-tightly attached to the lower end of the tubular container 2.

The tubular container 2 has in its upper end the opening part with which the cap 4 for the opening can normally be mesh-engaged and, in its lower end, has the portion whose inner diameter is slightly larger than the outer diameter of the stopper 3a. Further, the adapter 5a can be internally inserted into the lower end portion.

In the tubular container 2, the slidable stopper 3a is inserted in the vicinity of its lower end, and the cap 4 for the opening is detachably attached to its upper end. By the fact that the stopper 3a and the cap 4 for the opening are attached, the dissolving solution compartment 7 is formed in the tubular container 2. In the dissolving solution compartment 7, there is filled the liquid dissolving the specimen (nasal cavity wiping liquid, nasal cavity absorption liquid, pharynx wiping liquid, or the like). Usually, the dissolving solution is the water, and the surfactant is added in order to raise the reactivity with the specimen when the dissolving solution is dripped into the reaction reagent. Additionally, as the antiseptic, there is added sodium azide, or the like.

In order to push out the sampling liquid treated in the tubular container 2, there is not limited to the fact that the volume of the dissolving solution compartment is made relatively small compared to the dissolving solution quantity by pressing the tubular container 2 from the outside to thereby deform the tube part of the tubular container 2, and it may be pushed out by other physical means, or there may be performed such an operation as to raise the internal pressure by compressing the gas existing in the dissolving solution compartment.

Accordingly, it is desirable that the tubular container 2 has such a transparency that the state in the container can be confirmed and it does not become white even if repeatedly pressed, and the flexibility and, as a material like this, there is suitably used polyethylene, polypropylene, vinyl chloride resin, or the like.

Since the stopper 3a and the cap 4 for the opening are assembled while keeping the fluid-tightness of the dissolving solution compartment of the tubular container 2, there is no risk that the dissolving solution leaks from the tubular container 2 to the outside.

The stopper 3a has generally the shape of such a degree that there is no fact that it is distorted to thereby impair the fluid-tightness at the manufacturing time or at the long preservation time, desirably the cylindrical shape whose length (dimension) is small, and, before the specimen is added, it closely contacts with the inner wall of the tubular container 2. However, if the stopper 3a is slid and moved to the lower end direction of the tubular container 2, the gap is formed between it and the inner wall. There is made such that this gap is not closed even when the deformation of the dissolving solution compartment of the tubular container is released.

As the material of the stopper 3a, although there is enumerated the elastic material such as normal butyl rubber, halogenated butyl rubber, butadiene rubber, isoprene rubber, isobutylene rubber, or thermoplastic elastomer, usually the thermoplastic elastomer is used.

Further, for the purpose of smoothening the sliding, the annular rib may be provided in the stopper 3 so as to reduce a contact area with the inner wall.

The cap 4 for the opening is detachably attached to the tubular container 2, and usually mesh-engaged. Further, in order to retain the fluid-tightness, there is usually provided the sleeve jointing with the opening end inner wall of the tubular container.

The adapter 5a is the tubular member connected to the lower end of the tubular container 2, accommodates the stopper 3 pushed down when the dissolving solution compartment 7 of the tubular container 2 is pressed, forms the gap between the lower end inner wall of the tubular container 2 and the outer face of the stopper 3, and additionally possesses in its lower end the drip port 8. Before the sampling liquid is prepared, usually only the air exists in the adapter 5.

If the adapter 5a is attached to the tubular container 2 while retaining the fluid-tightness, it may be fusion-bonded, or bonded by the solvent, or additionally bonded under the pressed-in state. As the material like this, there is used polypropylene, ABS resin, polystylene, BS resin, polycarbonate resin, or the like, whose hardness is relatively higher than the tubular container (or whose bending elastic modulus is high).

In the lumen of the adapter 5a, a filter 6a is provided above the drip port 8 while adjoining it. The filter 6a is one which, in the case where the specimen collected from the patient is one having the high viscosity or contains the solid matter, has the bore diameter of such a degree that it is trapped such that the drip port does not clog. The filtration effective area and the coarseness of the pore can be freely selected. As the material like this, besides polyethylene, polypropylene, fluorine-based resin, nylon resin, or the like, although there is used the inorganic material or the like, usually there is used the polyethylene sintered body.

In a drip port 8a, there is provided the cap 9 for the drip port, which is detachably attached. The cap 9 for the drip port may be detachably attached to the drip port 8a and, as the material, there is selected a material softer than the material used in the adapter, and generally there is used polyethylene, polypropylene, vinyl chloride resin, or the like. Further, it may be formed monolithically with the adapter 5a. Additionally, in order that the plural sampling liquid containers can be stood in the exclusive container stand, the diameter of the exclusive container stand may be made the dimension approximately the same as the barrel diameter of the tubular container 2.

It is desirable that the cap 4 for the opening and/or the cap 9 for the drip port have/has in each top face a top plate and a peripheral wall vertically provided while continuing to the top plate and, in an outer periphery face of the peripheral wall, there are subsidiarily provided irregularities for preventing a slip when rotating the cap (4, 9).

In the present invention, there is characterized by having a knob part 41 not protruding in an external direction from the outer periphery face provided in the top face of the cap 4 for the opening. Further, in the present invention, also the cap 9 for the drip port has a knob part 91. The knob parts 41 and 91 have such lengths and widths as to be easy to exert a force like torque especially in a drawing-out direction and a rotation direction in order to smoothly perform an attachment to and a det achment from the tubular container 2 and the adapter 5. A dimension of the knob part can be freely set while matching for a combination with a reagent container and a swab, which are packed together, and a size of an accommodation box. Further, in the knob parts 41 and 91, concave grooves or convex ribs may be provided in a perpendicular direction to an attachment/detachment direction in order to prevent fingers from slipping and improve an operability. Or, an embossing may be applied. Besides these workings, there may be made a contrivance in order to facilitate the fingers to be applied from any direction by extending an auxiliary means so as to intersect like a cross, or the like. As a material of the cap 4 for the opening, in which there is provided the knob part 41 like this, usually there is suitably used polypropylene, ABS resin, polycarbonate, cyclic polyolefin, BS resin, or the like, whose hardness is relatively higher than the tubular container (or whose bending elastic modulus is high).

In the present invention, by the fact that there is provided the auxiliary means taking out the sampling liquid container, which extends in the upper end and/or the lower end of the container concerned, the technician can simply take out the sampling liquid container from the accommodation box, and it is possible to prevent the risk in the contamination and the infection by the virus (refer to Fig. 8).

When preparing the sampling liquid, first the cap 4 for the opening of the opening part is released under the state directed upward, the sampling rod, such as swab, to which there adheres the specimen collected from the patient is inserted into the dissolving solution compartment of the tubular container 2 from the upper end opening part, and the specimen is dissolution-extracted by the dissolving solution, thereby preparing the sampling liquid.

Next, the cap 4 for the opening is attached again to the opening part of the tubular container 2 and, after the drip port 8 placed in the side opposite to the tubular container 2 is directed to the reaction reagent container, if the barrel part of the tubular container 2, in which the dissolving solution compartment exists, is pressed, the stopper 3a advances downward by the pressure rise in the inside due to that pressing pressure, and moves into the adapter 5a and is accommodated. Between the outer face of the accommodated stopper 3a and the inner wall of the adapter 5a, there is provided the gap through which the sampling liquid in the dissolving solution compartment 7 can pass and drip. Accordingly, the sampling liquid passes through this gap, is guided from the drip port 8 to the outside, and can drip to the reaction reagent container.

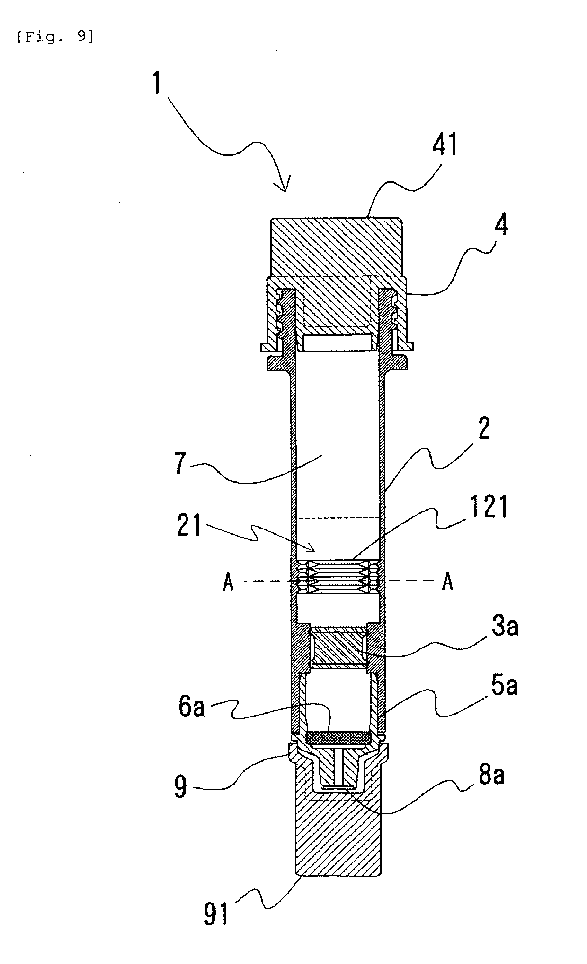

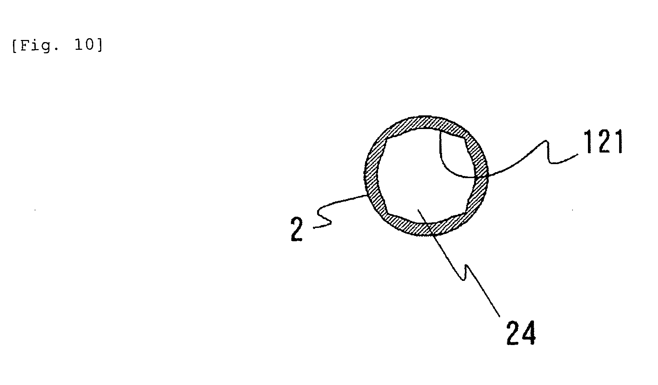

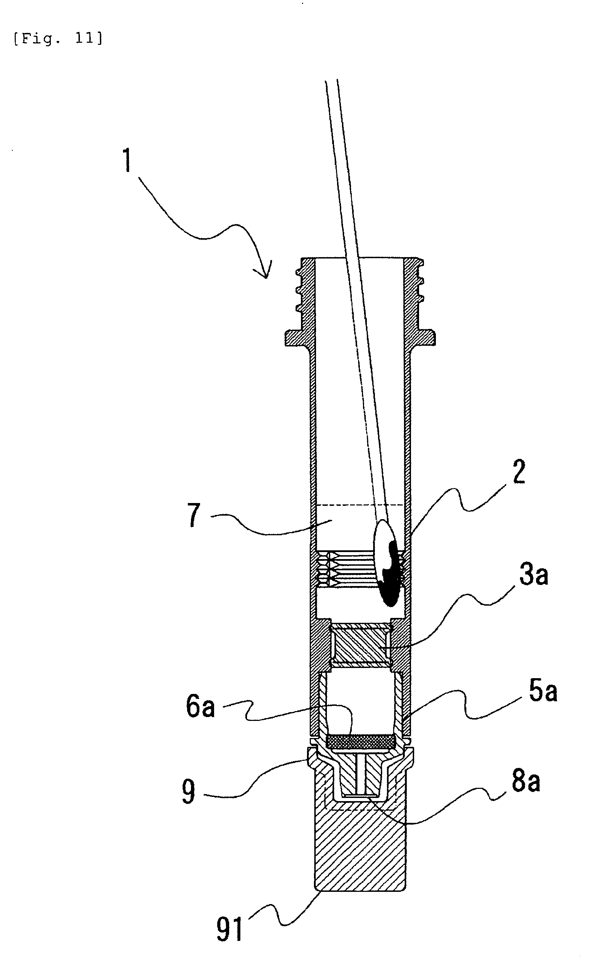

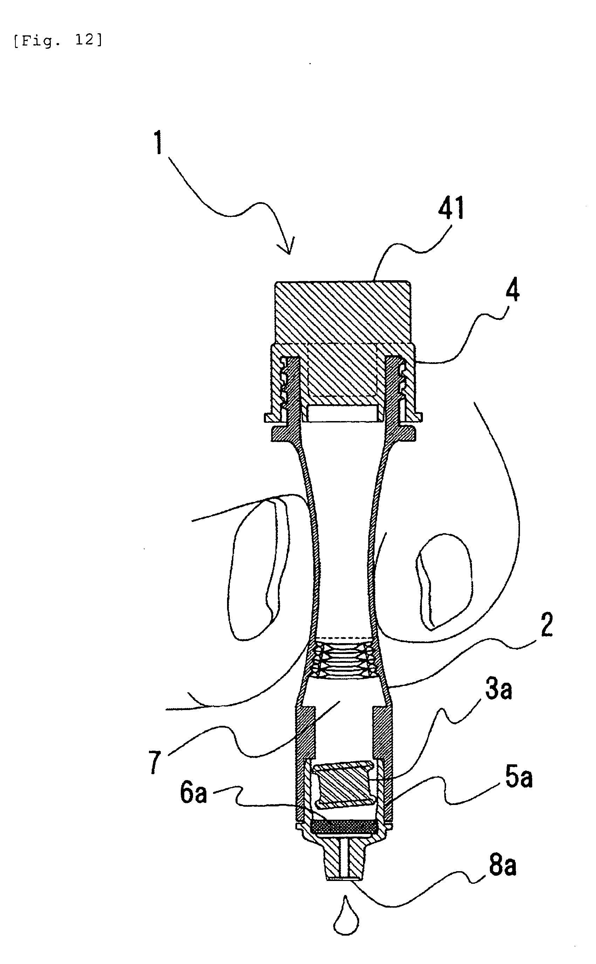

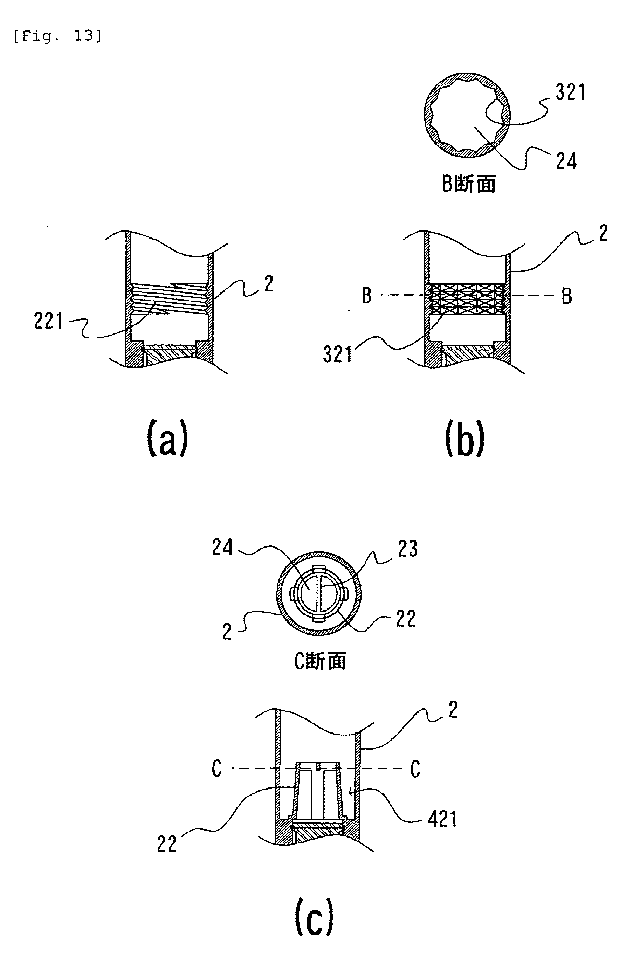

Other sampling liquid container concerned with the present invention is concretely explained by using the drawings. Fig. 9 is a longitudinal sectional view of the sampling liquid container of the present invention, and Fig. 10 is a sectional view of A - A in Fig. 9. Fig. 11 is a longitudinal sectional view showing a state in which the swab which collected the specimen is inserted from the opening part of the sampling liquid container of Fig. 9. Fig. 12 is a schematic view showing a state in which the dissolving solution compartment 7 of the tubular container of the sampling liquid container of Fig. 9 is pressed from the outside, the stopper is pushed down, and the sampling liquid passes through the gap between the inner face of the tubular container and the outer face of the stopper and drips from the drip port. Figs. 13(a) - (c) are sectional views showing other embodiments of the auxiliary means for promoting a dissolution or an extraction of a specimen sample in the sampling liquid container of the present invention.

As shown in Fig. 11, the sampling liquid container 1 of the present invention has the tubular container 2 opening in its upper end and lower end, the stopper 3a slidably inserted into the tubular container 2 in order to provide the fluid-tight dissolving solution compartment 7 in the tubular container, and the cap 4 detachably attached to the upper end of the tubular container 2, and has the adapter 5a fluid-tightly attached to the lower end of the tubular container 2.

The tubular container 2 has in its upper end the opening part with which the cap 4 can be normally mesh-engaged and, in its lower end, has the portion whose inner diameter is slightly larger than the outer diameter of the stopper 3a. Further, the adapter 5a can be internally inserted into the lower end portion. In the tubular container 2, the slidable stopper 3a is inserted in the vicinity of its lower end, and the cap 4 is detachably attached to its upper end. By the fact that the stopper 3a and the cap 4 are attached, the dissolving solution compartment 7 is formed in the tubular container 2. In the dissolving solution compartment 7, there is filled the liquid dissolving the specimen (nasal cavity wiping liquid, nasal cavity absorption liquid, pharynx wiping liquid, or the like). Usually, the dissolving solution is the water, and the surfactant is added in order to raise the reactivity with the specimen when the dissolving solution is dripped into the reaction reagent. Additionally, as the antiseptic, there is added sodium azide, or the like.

In order to push out the sampling liquid treated in the tubular container 2, there is not limited to the fact that the volume of the dissolving solution compartment 7 is made relatively small compared to the dissolving solution quantity by pressing the tubular container 2 from the outside to thereby deform the tube part of the tubular container 2, and it may be pushed out by other physical means, or there may be performed such an operation as to raise the internal pressure by compressing the gas existing in the dissolving solution compartment 7. Accordingly, it is desirable that the tubular container 2 has such a transparency that the state in the container can be confirmed and it does not become white even if repeatedly pressed, and the flexibility and, as a material like this, there is suitably used polyethylene, polypropylene, vinyl chloride resin, or the like.

The swab here indicates a wound cotton element for medical care, is concretely an instrument in which a nonwoven fabric or the like is attached to a tip of a plastic-made or paper-made shaft like a rod, and one for collecting a humor sample and, as a material of the nonwoven fabric, there is used rayon, paper, polylactic acid, cotton fiber, or the like.

In the present invention, it is a characteristic that there is provided an auxiliary means 21 for promoting the dissolution or the extraction of the specimen sample in the dissolving solution compartment 7 and, as the auxiliary means 21, there is a separation wall formed with the tubular container 2 being protruded to an inside and with a through-hole 24 for inserting the swab being provided in a center part. As the separation wall like this, concretely there is annular protrusions 121 (Fig. 9, Fig. 10) continuously provided in a axial direction while being provided on an inner wall of the tubular container 2, or a spiral protrusion 221 (Fig. 13(a)) provided on the inner wall of the tubular container 2, or protrusions 321 (Fig. 13 (b) ) separated on the inner wall of the tubular container 2 with a predetermined interval.

As shown in Fig. 9, it is desirable that plural annular protrusions 121 continuously provided in an axial direction are on the inner wall rather than being in a whole face of the inner wall, thereby leaving between the respective protrusions a portion buffering a stiff feeling when the tubular container is squeezed. Although the annular protrusions shown in Fig. 9 comprise four protrusions, it is not limited to these. It is desirable that normally this annular protrusion is disposed in a position upwardly and downwardly straddling a liquid level while coinciding with a motion, in which a cotton ball moves up and down while straddling the liquid level, so as to easily scrape off the wiping liquid from the cotton ball. Additionally, it is desirable that there is spaced a distance by which a communication is not performed carelessly by the fact that the stopper is pushed by a tip of the swab. It is to be desired that a height of the protrusion is rather as low as possible so as to easily obtain an internal pressure rise when squeezed. Concretely, although it is desirable that this is normally about 0.1 - 0.3 mm, it can be freely selected in compliance with a dimension and a shape of the cotton ball so long as the wiping liquid can be scraped off.

Further, as shown in Fig. 13(a), it is desirable that the spiral protrusion 221 of about 2 - 5 stripes are provided on the inner wall face rather than being provided in the whole face of the inner wall. It is desirable that normally also this spiral protrusion is disposed in the position upwardly and downwardly straddling the liquid level while coinciding with the motion, in which the cotton ball moves up and down while straddling the liquid level, so as to easily scrape off the wiping liquid from the cotton ball. Additionally, it is desirable that there is spaced the distance by which the communication is not performed carelessly by the fact that the stopper is pushed by the tip of the swab. It is to be desired that the height of a ridge of the spiral is rather as low as possible so as to easily obtain the internal pressure rise when squeezed. Concretely, although it is desirable that this is normally about 0.1 - 0.3 mm, it can be freely selected in compliance with the dimension and the shape of the cotton ball so long as the wiping liquid can be scraped off.

Additionally, it is desirable that, as shown in Fig. 13(b), the protrusions 321 separated on the inner wall with the predetermined interval is provided on the inner wall face in a range straddling the liquid level when the tubular container is stood, and each protrusion has a height and an interval of such a degree that there is no stiff feeling when squeezed and a content can be visually confirmed from the outside. It is desirable that normally this protrusion spaced with the predetermined interval is disposed in the position upwardly and downwardly straddling the liquid level while coinciding with the motion, in which the cotton ball moves up and down while striding over the liquid level, so as to easily scrape off the wiping liquid from the cotton ball. Additionally, it is desirable that there is spaced the distance by which the communication is not performed carelessly by the fact that the stopper is pushed by the tip of the swab. It is to be desired that the height of the protrusion is rather as low as possible so as to easily obtain the internal pressure rise when squeezed. Concretely, although it is desirable that this is normally about 0.1 - 0.3 mm, it can be freely selected in compliance with the dimension and the shape of the cotton ball so long as the wiping liquid can be scraped off.

Further, as shown in Fig. 13(c), the auxiliary means 21 may be a protrusion 421 which has, on an upper end of a portion retaining the stopper 3 in the lower end of the tubular container 2, plural pillar parts 22 extending in a direction from the lower end to the upper end of the tubular container 2, and in an apex part of the pillar part 22, a beam 23 in a radial direction. By rubbing the swab against the beam 23, it becomes possible to scrape off a mucus matter adhered to the swab and, after a drop of the stopper 3, the sampling liquid flows through an interstice of the pillar part 22 or the beam 23 and moves to the filter 6. In order that it is easy to scrape off the wiping liquid from the cotton ball, this beam is normally disposed approximately on the liquid level while coinciding with the motion in which the cotton ball moves up and down while striding over the liquid level. Further, it is desirable that there is spaced the distance by which the communication is not performed carelessly by the fact that the stopper is pushed by the tip of the swab. Additionally, there is made such that, in a side part of the beam, there exists a space approximately the same as or slightly larger than the cotton ball such that a side part of the cotton ball impinges. A number and a direction of the beam can be freely selected in compliance with the dimension and the shape of the cotton ball so long as the wiping liquid can be scraped off.

Although the auxiliary means 21 is normally formed monolithically with the tubular container, it may be a separate body and incorporated. It is desirable that a material of the auxiliary means 21 in this case is formed by an elastic body, e.g., elastomer. A length of the auxiliary means 21 at this time is a length of such a degree that it is not disengaged by slanting when the tubular container 2 is squeezed, and may be freely set so long as there does not become an obstacle in visually confirming the content. The protrusion of the auxiliary means 21 makes an insertion of the swab into the tubular container 2 possible, and it is necessary that it has a through-hole 24 bringing about its free motion.

As shown in Fig. 11, when preparing the sampling liquid, first the cap 4 of the opening part is released under the state directed upward, the sampling rod, such as swab, to which there adheres the specimen collected from the patient is inserted into the dissolving solution compartment of the tubular container 2 from the upper end opening part, and the specimen is scraped off by moving up and down on the auxiliary means 21 to thereby be dissolution-extracted by the dissolving solution, thereby preparing the sampling liquid.

Next, as shown in Fig. 12, the cap 4 is attached again to the opening part of the tubular container 2 and, after the drip port 8a placed in the side opposite to the tubular container 2 is directed to the reaction reagent container, if the barrel part of the tubular container 2, in which the dissolving solution compartment 7 exists, is pressed, the stopper 3a advances downward by the pressure rise in the inside due to that pressing pressure, and moves into the adapter 5a and is accommodated. Between the outer face of the accommodated stopper 3a and the inner wall of the adapter 5a, there is provided the gap through which the sampling liquid in the dissolving solution compartment 7 can pass and drip. Accordingly, the sampling liquid passes through this gap, is guided from the drip port 8a to the outside, and can drip to the reaction reagent container.

In the present invention, as the filter, there is a sintered body, a foam-molded body, a nonwoven fabric, a fiber-laminated body, or the like. As the material like this, besides polyethylene, polypropylene, fluorinated resin, nylon resin, or the like, although there is used the inorganic material or the like, normally there is used the polyethylene sintered body.

In the present invention, the filter 6 is a continuous porous body whose pore diameter is about 0.1 µm - about 20 µm, desirably about 1.0 µm - about 10 µm, and whose thickness is about 1.0 mm - about 6.0 mm, desirably about 1. 5 mm - about 4.5 mm. The filter 6 may be one sheet or plural sheets. If the pore diameter of the filter is less than about 0.1 µm, in a case where the specimen collected from the patient is one of a high viscosity or contains the solid matter, there occurs a case where the drip port clogs. Further, if it exceeds about 20 µm, the sampling liquid becomes easy to pass through the filter, and becomes easy to spatter like the water gun. If the thickness is less than about 1. 0 mm, the sampling liquid existing in the dissolving solution compartment and the gas existing in the lumen of the adapter become easy to pass through the filter under a mixed state, and become easy to spatter like the water gun from the drip port. If it exceeds about 7.5 mm, since the filter becomes too complicated because a path for passing the liquid is long, the surfactant contained in the sampling liquid and the gas are mixed and become easy to generate foams, so that there becomes such that a mixing of the foams is conspicuous from a tip of the drip port.

It is desirable that the drip port 8 has a hollow groove whose bore diameter is about 0.5 mm - about 3.5 mm, desirably about 1.0 mm - about 3.3 mm, and whose length is about 6 mm - about 15 mm, desirably about 6 mm - about 12 mm. Additionally, a quantity of the liquid to be filled in the adapter 5 is normally about 0.1 mL - about 2.0 mL, desirably about 0.4 mL - about 1.5 mL.

In Fig. 4, the cap 9 for the drip port, which is detachably attached, is provided to the drip port 8a. The cap 9 for the drip port may be detachably attached to the drip port 8a and, as a material, there is used polyethylene, polypropylene, vinyl chloride resin, or the like. Further, it may be formed monolithically with the adapter 5a. Additionally, in order that the plural sampling liquid containers 1 can be stood in the exclusive container stand, the diameter of the exclusive container stand may be approximately the same size as the barrel diameter of the tubular container 2. The knob part 91 may be provided in the cap 9 for the drip port.

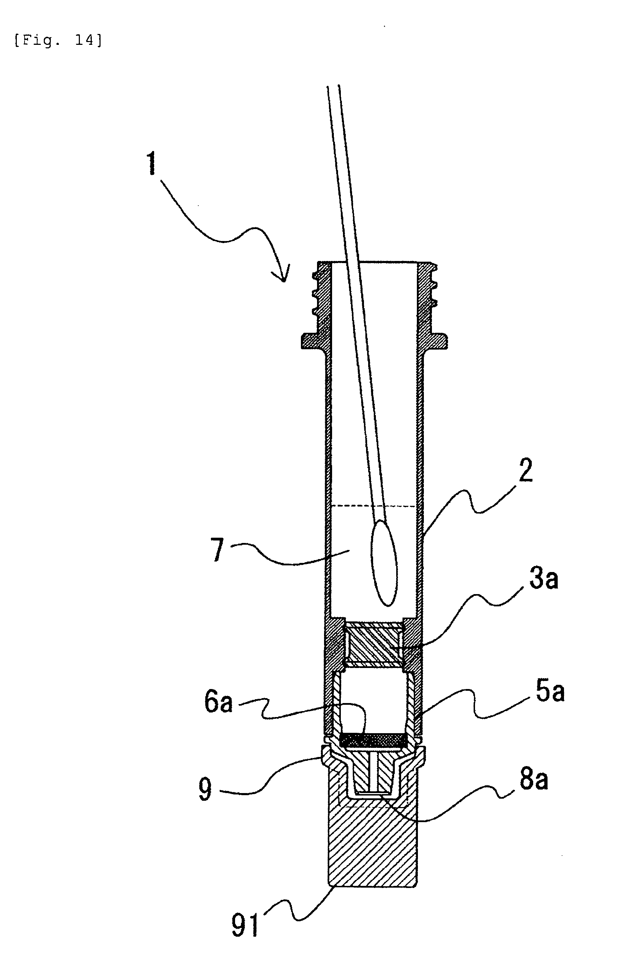

As shown in Fig. 14, when preparing the sampling liquid, first the cap 4 of the opening part is released under the state directed upward, the sampling rod, such as swab, to which there adheres the specimen collected from the patient is inserted into the dissolving solution compartment of the tubular container 2 from the upper end opening part, and the specimen is scraped off by moving up and down on the auxiliary means 21 (not shown in the drawing) to thereby be dissolution-extracted by the dissolving solution, thereby preparing the sampling liquid.

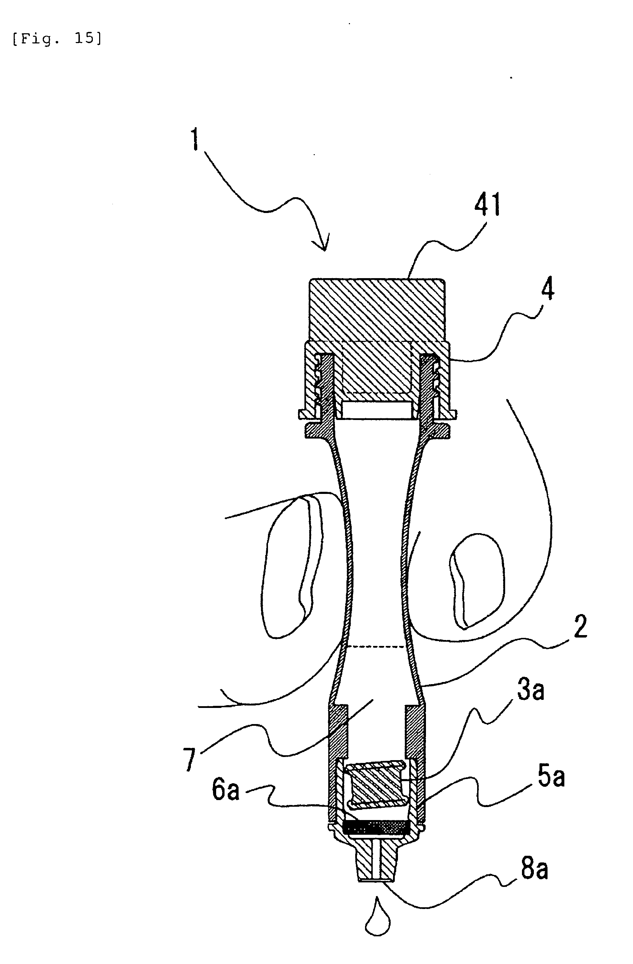

Next, as shown in Fig. 15, the cap 4 is attached again to the opening part of the tubular container 2 and, after the drip port 8a placed in the side opposite to the tubular container 2 is directed to the reaction reagent container, if the barrel part of the tubular container 2, in which the dissolving solution compartment 7 exists, is pressed, the stopper 3a advances downward by the pressure rise in the inside due to that pressing pressure, and moves into the adapter 5a and is accommodated. Between the outer face of the accommodated stopper 3a and the inner wall of the adapter 5a, there is provided the gap through which the sampling liquid in the dissolving solution compartment 7 can pass and drip. Accordingly, the sampling liquid passes through this gap, passes through the filter 6, is guided from the drip port 8a to the outside, and can drip to the reaction reagent container.

Hereunder, the present invention is explained in more detail by an embodiment.

### Embodiment 1

There were prepared the sampling liquid containers (Fig. 1) which possessed sintered filters (made by Filteren Company, high density polyethylene) of three kinds shown in Table 1, whose surface pore diameters were different. There was confirmed an existence/nonexistence of a water gun phenomenon at a liquid communication time by squeezing an outer part of the tubular container with the hand by variously changing (Tables 2 - 4) liquid-filled quantities (0.4 - 1.0 mL) of the dissolving solution compartment, and surface pore diameters (2.0 - 30 µm) and thicknesses (1.5 - 6.0 mm × 5 sheets) of the filter.

**[Table 1]**

| Sintered filter | | | |
|---|---|---|---|
| No. | Type | Surface pore diameter (µm) | Size (mm) |
| A | XHDPE (HDPE-made) | 2.0 | ϕ7.5 × 1.5 |
| B | UHDPE (HDPE-made) | 10 | ϕ7.5 × 1.5 |
| C | XUHDPE (HDPE-made) | 30 - 50 | ϕ7.5 × 1.5 |

**[Table 2]**

| Sample | Filter kind | Pore diameter (µm) | Thickness (mm) | Sheet number | Filled quantity (mL) | N number |
|---|---|---|---|---|---|---|
| A-1 | XHDPE | 2 | 1.5 | 1 | 1.0 | 5 |
| A-2 | XHDPE | 2 | 3.0 | 2 | 1.0 | 5 |
| A-3 | XHDPE | 2 | 4.5 | 3 | 1.0 | 5 |
| B-1 | UHDPE | 10 | 1.5 | 1 | 1.0 | 5 |
| B-2 | UHDPE | 10 | 3.0 | 2 | 1.0 | 5 |
| B-3 | UHDPE | 10 | 4.5 | 3 | 1.0 | 5 |
| B-4 | UHDPE | 10 | 6.0 | 4 | 1.0 | 5 |
| C-1 | XUHDPE | 30 | 1.5 | 1 | 1.0 | 5 |
| C-2 | XUHDPE | 30 | 3.0 | 2 | 1.0 | 5 |
| C-3 | XUHDPE | 30 | 4.5 | 3 | 1.0 | 5 |

**[Table 3]**

| Sample | Filter kind | Pore diameter (µm) | Thickness (mm) | Sheet number | Filled quantity (mL) | N number |
|---|---|---|---|---|---|---|
| A-11 | XHDPE | 2 | 1.5 | 1 | 0.7 | 5 |
| A-12 | XHDPE | 2 | 3.0 | 2 | 0.7 | 5 |
| A-13 | XHDPE | 2 | 4.5 | 3 | 0.7 | 5 |
| B-11 | UHDPE | 10 | 1.5 | 1 | 0.7 | 5 |
| B-12 | UHDPE | 10 | 3.0 | 2 | 0.7 | 5 |
| B-13 | UHDPE | 10 | 4.5 | 3 | 0.7 | 5 |
| B-14 | UHDPE | 10 | 6.0 | 4 | 0.7 | 5 |
| C-11 | XUHDPE | 30 | 1.5 | 1 | 0.7 | 5 |
| C-12 | XUHDPE | 30 | 3.0 | 2 | 0.7 | 5 |
| C-13 | XUHDPE | 30 | 4.5 | 3 | 0.7 | 5 |

**[Table 4]**

| Sample | Filter kind | Pore diameter (µm) | Thickness (mm) | Sheet number | Filled quantity (mL) | N number |
|---|---|---|---|---|---|---|
| A-21 | XHDPE | 2 | 1.5 | 1 | 0.4 | 5 |
| A-22 | XHDPE | 2 | 3.0 | 2 | 0.4 | 5 |
| A-23 | XHDPE | 2 | 4.5 | 3 | 0.4 | 5 |
| B-21 | UHDPE | 10 | 1.5 | 1 | 0.4 | 5 |
| B-22 | UHDPE | 10 | 3.0 | 2 | 0.4 | 5 |
| B-23 | UHDPE | 10 | 4.5 | 3 | 0.4 | 5 |
| B-24 | UHDPE | 10 | 6.0 | 4 | 0.4 | 5 |
| C-21 | XUHDPE | 30 | 1.5 | 1 | 0.4 | 5 |
| C-22 | XUHDPE | 30 | 3.0 | 2 | 0.4 | 5 |
| C-23 | XUHDPE | 30 | 4.5 | 3 | 0.4 | 5 |

Results in which the water gun phenomenon is confirmed are shown in Table 5. In Table 5, a case where the water gun phenomenon from the drip port tip is not seen at the liquid communication time is displayed as a mark O, a case where although the water gun phenomenon from the drip port tip does not exist at the liquid communication time, several drops appear while delaying if a squeeze state is retained as a mark Δ, and a case where the liquid appears from the drip port like the water gun at the liquid communication time as a mark x.

**[Table 5]**

| Sample | Confirmation test (N = 5) | Evaluation | Sample | Confirmation test (N = 5) | Evaluation | Sample | Confirmation test (N = 5) | Evaluation |
|---|---|---|---|---|---|---|---|---|
| A-1 | ○○○○○ | Suitable | A-11 | ○○○○△ | Suitable | A-21 | △○△△○ | Suitable |
| A-2 | ○○○○○ | Suitable | A-12 | ○○○○○ | Suitable | A-22 | ○○○○○ | Suitable |
| A-3 | ○○○○○ | Suitable | A-13 | ○○○○○ | Suitable | A-23 | ○○○○○ | Suitable |
| B-1 | ○○○○○ | Suitable | B-11 | ×○×○× | Unsuitable | B-21 | ××××× | Suitable |
| B-2 | ○○○○○ | Suitable | B-12 | △○△×○ | Unsuitable | B-22 | △○○×△ | Suitable |
| B-3 | ○○○○○ | Suitable | B-13 | ×○○×△ | Unsuitable | B-23 | ××△×△ | Suitable |
| B-4 | ○○○○○ | Suitable | B-14 | △△○○○ | Suitable | B-24 | △×××× | Suitable |
| C-1 | ○○○×○ | Unsuitable | C-11 | ○××○× | Unsuitable | C-21 | ××××× | Unsuitable |
| C-2 | ○×○○○ | Unsuitable | C-12 | △△×△△ | Unsuitable | C-22 | ××××× | Unsuitable |
| C-3 | △○○○○ | Suitable | C-13 | △△×△△ | Unsuitable | C-23 | ××××× | Suitable |

As obvious from Table 5, if there is selected the filter (Sample A) of 2 µm in the pore diameter and the thickness (1.5 mm × 5 sheets = 7.5mm), it is possible to prevent the water gun phenomenon irrespective of the liquid-filled quantity. Further, if the pore diameter becomes 10 µm (Sample B), it is necessary that the liquid-filled quantity is at least no less than 1.0 mL. Additionally, in the pore diameter 30 µm (Sample C), the water gun phenomenon occurs irrespective of the liquid-filled quantity.

The sampling liquid container of the present invention is not limited to the test of the virus such as influenza, and can be utilized in a general test of an organism containing other pathogen.

## Claims

1. A sampling liquid container having a tubular container opening in its upper end and lower end, a stopper slidably inserted into the tubular container in order to provide a fluid-tight dissolving solution compartment in the tubular container, and a cap for the opening, which is detachably attached to the upper end of the tubular container, and having an adapter fluid-tightly attached to the lower end of the tubular container, **characterized in that** an inner diameter of the lower end of the tubular container is slightly larger than an outer diameter of the stopper, the adapter accommodates the stopper pushed down when the dissolving solution compartment of the tubular container is pressed, a gap is formed between a lower end inner wall of the tubular container and an outer face of the stopper, and additionally a drip port is possessed in the lower end.

2. A sampling liquid container according to claim 1, wherein a filter is provided in a lumen of the adapter above the drip port while adjoining it.

3. A sampling liquid container according to claim 1 or 2, wherein there is provided a cap for the drip port, which is detachably attached to the drip port.

4. A sampling liquid container according to claim 1, wherein additionally, in the upper end and/or the lower end of the sampling liquid container, there is provided an auxiliary means taking out the container.

5. A sampling liquid container according to claim 4, wherein the auxiliary means taking out the container is a knob part not protruding in an external direction from an outer periphery face of the sampling liquid container.

6. A sampling liquid container according to claim 4 or 5, wherein the auxiliary means taking out the container is provided in a top face of the cap for the opening.

7. A sampling liquid container according to claim 4, wherein the drip port is a drip port for discharging the sampling liquid in which a specimen sample is introduced into a dissolving solution in the lower end.

8. A sampling liquid container according to claim 7, wherein the drip port is provided in the adapter attached to a lower end part of the tubular container.

9. A sampling liquid container according to claim 7, wherein a cap for the drip port is provided to the drip port, and the auxiliary means taking out the container is provided in the cap for the drip port.

10. A sampling liquid container according to claim 9, wherein the cap for the opening and/or the cap for the drip port have/has in each top face a top plate and a peripheral wall vertically provided while continuing to the top plate and, in an outer periphery face of the peripheral wall, there are provided irregularities for preventing a slip.

11. A sampling liquid container according to claim 1, wherein additionally an auxiliary means for promoting a dissolution or an extraction of a specimen sample in the dissolving solution compartment is provided in an inside of the tubular container.

12. A sampling liquid container according to claim 11, wherein the auxiliary means for promoting the dissolution or the extraction of the specimen sample is a separation wall formed with the tubular container being protruded inward and a through-hole into which a swab is inserted being provided in a center part.

13. A sampling liquid container according to claim 12, wherein the separation wall is annular protrusions continuously provided in an axial direction, which is provided on an inner wall of the tubular container.

14. A sampling liquid container according to claim 12, wherein the separation wall is a spiral protrusion provided on an inner wall of the tubular container.

15. A sampling liquid container according to claim 12, wherein the separation wall is protrusions separated on an inner wall of the tubular container with a predetermined interval.

16. A sampling liquid container according to claim 11, wherein the auxiliary means for promoting the dissolution or the extraction of the specimen sample is a protrusion having plural pillar parts extending in a direction from the lower end to the upper end of the tubular container and possessing a beam in a radial direction in an apex part of the pillar part.

17. A sampling liquid container according to claim 1, wherein a filter is provided in an inner tube part of the adapter while adjoining the drip port, and the filter is a continuous porous body whose pore diameter is about 0.1 µm - about 20 µm, and whose thickness is about 1.0 mm - about 6.0 mm.

18. A sampling liquid container according to claim 1, wherein the drip port has a hollow groove of about 0.5 mm - about 2 mm in bore diameter, and about 6 mm - about 15 mm in length.

19. A sampling liquid container according to claim 1, wherein a quantity of a dissolving solution filled in the dissolving solution compartment is about 0.1 - 2.0 mL.

20. A sampling liquid container according to claim 1, wherein the filter is a sintered body, a foam-molded body, a nonwoven fabric, or a fiber-laminated body.
